# EUROPEAN PATENT SPECIFICATION

(11) **EP 4 114 482 B1**
(45) Date of publication and mention of the grant of the patent: **29.10.2025**
(21) Application number: 21712692.9
(22) Date of filing: 25.02.2021
(51) Int. Cl.: A61M 5/14, A61M 5/142, A61M 39/08

(54) **INFUSION CONNECTION LINE**
INFUSIONSANSCHLUSSLEITUNG
LIGNE DE RACCORDEMENT DE PERFUSION

(30) Priority: 02.03.2020 US 202016806757
(43) Date of publication of application: 11.01.2023
(73) Proprietor: CareFusion 303, Inc., San Diego, CA 92130 (US)
(72) Inventor: ZHU, Steven, San Diego, California 92130 (US)
(74) Representative: Epping - Hermann - Fischer
(86) International application number: PCT/US2021/019704
(87) International publication number: WO 2021/178220

(56) References cited:
- US-A- 4 798 590
- US-A1- 2005 277 890
- US-A1- 2006 173 412
- US-A1- 2012 053 557

## Description

### BACKGROUND

Intravenous (IV) infusion sets are used in the medical field for inserting liquid substances into a patient. IV infusion sets are typically constructed by joining multiple translucent polymeric tubing segments to multiple polymeric components. IV infusion sets for use with infusion pumps are typically dedicated to infusion pump use as a complete infusion set. Further, a dedicated infusion set is required for each brand and type of infusion pump, where a particular dedicated infusion set is not compatible with any other brand or type of infusion pump. The need to buy and stock multiple different dedicated IV infusion sets results in high costs to the health care industry.

Document US 2005/277890 A1 discloses a medical delivery system that is provided for delivering a medicament or fluid to a patient. The system comprises a disposable element such as a line set associated with a container containing the fluid, an identifier associated with the line set and having identification information associated therewith, and a delivery device configured to engage the line set and deliver the fluid to the patient

Document US 2012/053557 A1 discloses a cassette is disclosed that comprises an interface element having a first position and a second position, the interface element configured to be driven from the first position to the second position and back to the first position by unidirectional rotary motion of a driving element.

The documents US 2006/173412 A1 and US 4 798 590 A disclose further medical infusion devices.

In view of the high costs of dedicated pump infusion sets, a typical work around in the health care industry is to substitute a gravity IV set for use with an infusion pump. However, gravity IV sets are not rated or qualified for use with infusion pumps, resulting in inaccuracies during pump infusion. Gravity IV sets also pose a much higher risk for broken tubing during pump infusion, which may result in patient injury. It is desirable to provide IV infusion sets for infusion pumps with reduced costs and risks.

### SUMMARY

The present disclosure provides IV infusion sets having an infusion connection line for insertion into infusion pumps.

The present invention is directed to an infusion connection line according to independent claim 1. The infusion connection line includes a pump infusion tube, a first connector coupled to a first end of the pump infusion tube and a second connector coupled to a second end of the pump infusion tube. The infusion connection line also includes an indicator element disposed between the first and second connectors. A portion of the pump infusion tube is configured to be disposed within an infusion pump, the infusion pump being any of a plurality of types of infusion pumps used in the health care industry. The infusion connection line is configured to couple a gravity IV infusion set to the infusion pump.

In one or more aspects, the first connector is a female luer connector. In one or more aspects, the first connector is coupled to a first connector cap. In one or more aspects, the first connector is coupled to a female stop cock assembly. In one or more aspects, the first connector is coupled to a male stop cock assembly. In one or more aspects, the first connector is coupled to a needle free connector. In one or more aspects, the first connector is coupled to the gravity IV infusion set. In one or more aspects, the second connector is a male luer connector. In one or more aspects, the second connector is coupled to a second connector cap. In one or more aspects, the second connector is coupled to an extension infusion line, the extension infusion line configured to deliver a fluid from the infusion pump to a receiving entity. In one or more aspects, the pump infusion tube has a wall thickness of 0.45 mm to 0.65 mm. In one or more aspects, the pump infusion tube has a length of 300 mm to 400 mm. In one or more aspects, the pump infusion tube is formed of a high elasticity material comprising one of silicone and thermoplastic polyurethane.

In one or more embodiments, an infusion set is provided. The infusion set includes an infusion connection line. The infusion connection line includes a pump infusion tube, a female luer connector disposed on a first end of the pump infusion tube, a male connector disposed on a second end of the pump infusion tube and an indicator element disposed on the pump infusion tube between the female and male connectors. The infusion set also includes a gravity IV set coupled to the pump infusion tube. A portion of the infusion connection line is configured to be disposed within an infusion pump, the infusion pump being any of a plurality of types of infusion pumps used in the health care industry.

In one or more aspects, the infusion set includes a female stop cock assembly coupled to the female luer connector of the pump infusion tube, wherein the gravity IV set is coupled to the female stop cock assembly. In one or more aspects, the infusion set includes a male stop cock assembly coupled to the female luer connector of the pump infusion tube, wherein the gravity IV set is coupled to the male stop cock assembly. In one or more aspects, the infusion set includes a needleless connector coupled to the female luer connector of the pump infusion tube, wherein the gravity IV set is coupled to the needleless connector. In one or more aspects, the infusion set includes an extension infusion line coupled to the male connector of the pump infusion tube, the extension infusion line configured to deliver a fluid from the infusion pump to a receiving entity. In one or more aspects, the pump infusion tube has a wall thickness of 0.45 mm to 0.65 mm, and wherein the pump infusion tube has a length of 300 mm to 400 mm. In one or more aspects, the pump infusion tube is formed of a high elasticity material comprising one of silicone and thermoplastic polyurethane.

Additional features and advantages of the disclosure will be set forth in the description below and, in part, will be apparent from the description or may be learned by practice of the disclosure. The objectives and other advantages of the disclosure will be realized and attained by the structure particularly pointed out in the written description and claims hereof as well as the appended drawings.

It is to be understood that both the foregoing general description and the following detailed description are exemplary and explanatory and are intended to provide further explanation of the disclosure as claimed.

### BRIEF DESCRIPTION OF THE DRAWINGS

The accompanying drawings, which are included to provide further understanding of the disclosure and are incorporated in and constitute a part of this specification, illustrate embodiments of the disclosure and together with the description serve to explain the principles of the disclosure.
FIG. 1 depicts a schematic view of an example gravity IV set.
FIG. 2 depicts an example infusion pump.
FIG. 3 depicts an infusion connection line, according to some aspects of the disclosure.
FIG. 4 depicts an infusion connection line, according to some aspects of the disclosure.
FIG. 5 depicts an infusion connection line, according to some aspects of the disclosure.
FIG. 6 depicts an infusion connection line, according to some aspects of the disclosure.
FIG. 7 depicts an infusion connection line, according to some aspects of the disclosure.
FIG. 8 depicts an infusion connection line, according to some aspects of the disclosure.

### DETAILED DESCRIPTION

The detailed description set forth below describes various configurations of the subject technology and is not intended to represent the only configurations in which the subject technology may be practiced. The detailed description includes specific details for the purpose of providing a thorough understanding of the subject technology. Accordingly, dimensions are provided in regard to certain aspects as non-limiting examples. However, it will be apparent to those skilled in the art that the subject technology may be practiced without these specific details. In some instances, well-known structures and components are shown in block diagram form in order to avoid obscuring the concepts of the subject technology.

It is to be understood that the present disclosure includes examples of the subject technology and does not limit the scope of the appended claims. Various aspects of the subject technology will now be disclosed according to particular but non-limiting examples. Various embodiments described in the present disclosure may be carried out in different ways and variations, and in accordance with a desired application or implementation.

IV infusion sets may be formed from any combination of infusion components and tubing. Typically, the infusion components and tubing are fixedly connected together to form a disposable infusion set that is used once and then discarded. The infusion components and tubing may be formed from any suitable material (e.g., plastic, silicone, rubber).

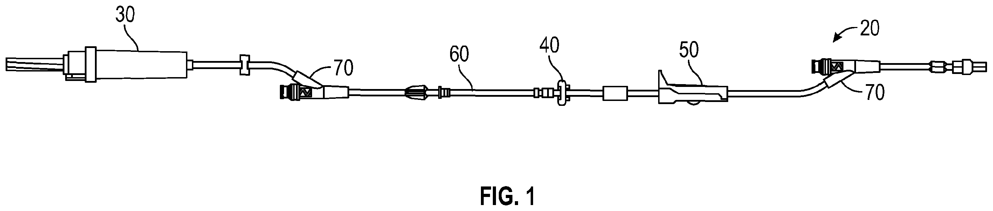

As shown in FIG. 1, an example gravity IV infusion set 20 may include a drip chamber 30, a check valve 40 and a roller clamp 50 connected together by tubing 60. Infusion set 20 may also include a Y-site 70 having a Y-shaped junction with an inlet port 72 connected to an inlet tube 62, a needleless connector 75 and an outlet port 74 connected to an outlet tube 64. The needleless connector 75 provides a port for the introduction of fluid into and/or removal of air bubbles from the infusion set 20. For example, a needleless syringe (not shown) may be connected to the needleless connector 75 in order to add medication to a saline fluid that is gravity fed from a saline bag (not shown) into the drip chamber 30. The infusion set 20 may include additional infusion components and may be formed of any combination of components and the tubing 60.

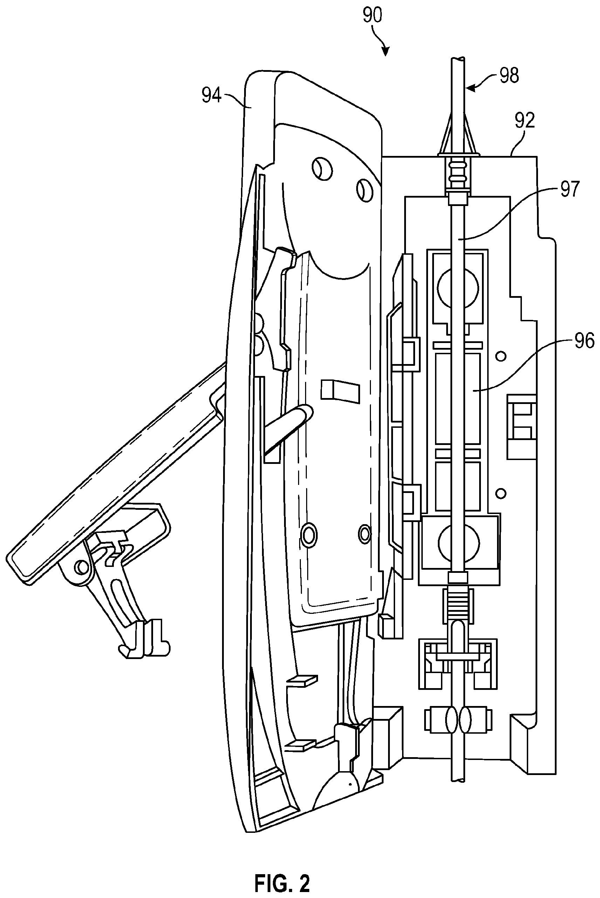

FIG. 2 shows an example infusion pump 90. The infusion pump 90 has a body 92 and a door 94. A tubing pathway 96 is disposed in the body 92. To use the infusion pump, the door 94 is opened and a tubing section 97 of an infusion set 98 is placed in the tubing pathway 96 and the door 94 is closed. The infusion pump 90 then engages and manipulates the tubing section 97 to cause fluid to be pumped through the infusion set 98.

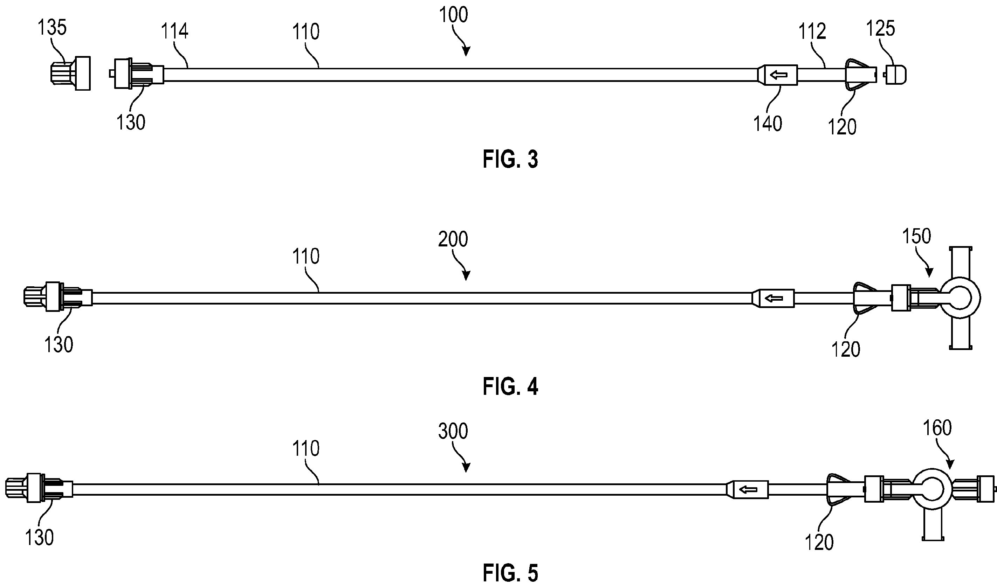

As shown in FIG. 3, an infusion connection line 100 is provided according to some aspects of the disclosure. Infusion connection line 100 includes a pump infusion tube 110. A first end 112 of the pump infusion tube 110 is coupled to a first connector 120, such as a female luer connector, for example. The first connector 120 may be coupled to a first connector cap 125, such as a female luer connector cap. A second end 114 of the pump infusion tube 110 is coupled to a second connector 130, such as a male luer lock, for example. The second connector 130 may be coupled to a second connector cap 135, such as a male luer lock cap.

An indicator element 140 is coupled to the pump infusion tube 110 to provide a visual indication for installation within an infusion pump. The indicator element 140 is formed/embedded within the material of the pump infusion tube 110. A portion of the pump infusion tube 110 between the second connector 130 and the indicator element 140 is configured to be inserted or disposed within an infusion pump (e.g., infusion pump 90). The indicator element 140 is configured to indicate the proper installation direction of the infusion connection line 100 within the infusion pump.

The pump infusion tube 110 may be formed of a high elasticity material, such as silicone or TPU, for example, which is configured to be manipulated by a compression mechanism of an infusion pump. The pump infusion tube 110 may have a thickness of 0.45-0.65 mm and may have a length of 300-400 mm. Thus, the pump infusion connection line 100 is configured to provide high accuracy and stable infusion delivery regardless of which brand or type of infusion pump is used.

As shown in FIG. 4, infusion connection line 200 may include additional connection assemblies according to some aspects of the disclosure. For example, infusion connection line 200 may be infusion connection line 100 without the first connector cap 125. Infusion connection line 200 also includes a female stop cock assembly 150 (e.g., 3-way female stop cock) coupled to the first connector 120. Further, infusion connection line 200 includes the same pump infusion tube 110 as infusion connection line 100. Accordingly, infusion connection lines 100 and 200 may be used in any brand or type infusion pump.

As shown in FIG. 5, infusion connection line 300 may include additional connection assemblies according to some aspects of the disclosure. For example, infusion connection line 300 may be infusion connection line 100 without the first connector cap 125. Infusion connection line 300 also includes a male stop cock assembly 160 (e.g., 3 way male stop cock) coupled to the first connector 120. Further, infusion connection line 300 includes the same pump infusion tube 110 as infusion connection lines 100, 200. Accordingly, infusion connection lines 100, 200 and 300 may be used in any brand or type infusion pump.

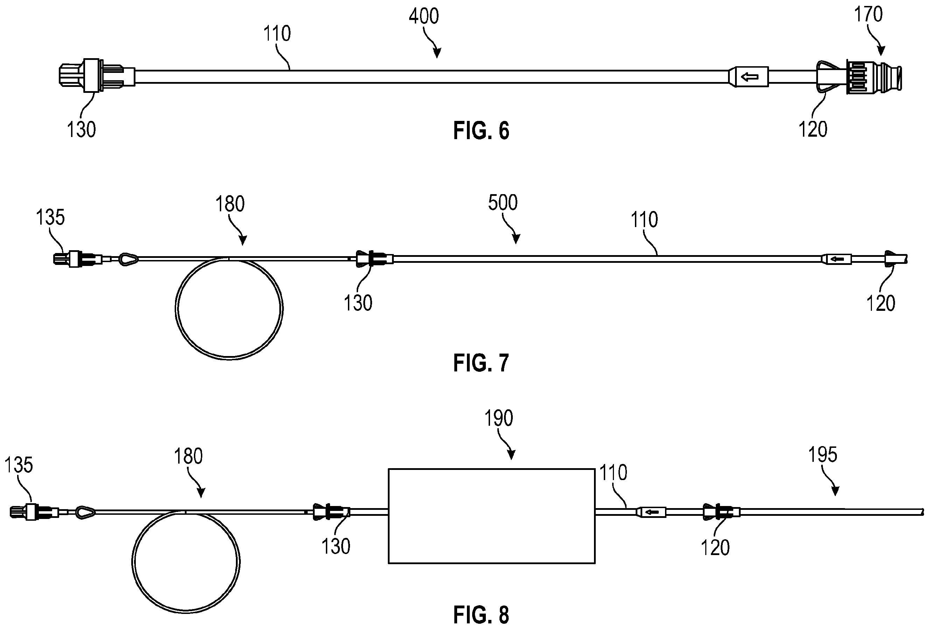

As shown in FIG. 6, infusion connection line 400 may include additional connection assemblies according to some aspects of the disclosure. For example, infusion connection line 400 may be infusion connection line 100 without the first connector cap 125. Infusion connection line 300 also includes a needle free connector 170 (e.g., needleless connector) coupled to the first connector 120. Further, infusion connection line 400 includes the same pump infusion tube 110 as infusion connection lines 100, 200, 300. Accordingly, infusion connection lines 100, 200, 300 and 400 may be used in any brand or type infusion pump.

As shown in FIG. 7, infusion connection line 500 may include additional connection assemblies according to some aspects of the disclosure. For example, infusion connection line 500 may be infusion connection line 100 without the second connector cap 135 coupled to the second connector 130. Infusion connection line 500 also includes an extension infusion line 180 coupled to the second connector 130, with the second connector cap 135 coupled to the extension infusion line 180. Further, infusion connection line 500 includes the same pump infusion tube 110 as infusion connection lines 100, 200, 300, 400. Accordingly, infusion connection lines 100, 200, 300, 400 and 500 may be used in any brand or type infusion pump.

As shown in FIG. 8, a gravity IV set 195 is coupled to infusion connection line 500 at the first connector 120. Pump infusion tube 110 is disposed within an infusion pump 190 (e.g., infusion pump 90 in FIG. 2). Thus, the gravity IV set 195 may be coupled to one or more fluid sources and a fluid flowing into the pump infusion tube 110 from the gravity IV set 195 is pumped into the extension infusion line 180 by the infusion pump 190. The extension infusion line 180 may be coupled to a receiving entity for receiving the fluid (e.g., an IV line for a patient). While infusion connection line 500 is shown to be engaged by infusion pump 190 in FIG. 8, it is understood that any of infusion connection lines 100, 200, 300, 400 may also be disposed within and/or engaged by infusion pump 190.

Because all of infusion connection lines 100, 200, 300, 400, 500 have the pump infusion tube 110 with first and second connectors 120, 130 in common, they may each be interchangeably used in any brand and type of infusion pump. Further, the configuration of the pump infusion tube 110 (e.g., material composition, thickness, size) provides for use in any brand and type of infusion pump while improving infusion accuracy and minimizing tube breakage and/or leakage in comparison to the infusion pump directly engaging a portion of a gravity IV set. In addition, the flexibility of coupling the base pump infusion tube 110 having first and second connectors 120, 130 to any of female stop cock assembly 150, male stop cock assembly 160, needle free connector 170, extension infusion line 180 and gravity IV set 195 provide for meeting a wide array of infusion needs using any infusion pump.

The present disclosure is provided to enable any person skilled in the art to practice the various aspects described herein. The disclosure provides various examples of the subject technology, and the subject technology is not limited to these examples. Various modifications to these aspects will be readily apparent to those skilled in the art, and the generic principles defined herein may be applied to other aspects.

A reference to an element in the singular is not intended to mean "one and only one" unless specifically so stated, but rather "one or more." Unless specifically stated otherwise, the term "some" refers to one or more. Pronouns in the masculine (e.g., his) include the feminine and neuter gender (e.g., her and its) and vice versa. Headings and subheadings, if any, are used for convenience only and do not limit the invention.

The word "exemplary" is used herein to mean "serving as an example or illustration." Any aspect or design described herein as "exemplary" is not necessarily to be construed as preferred or advantageous over other aspects or designs. In one aspect, various alternative configurations and operations described herein may be considered to be at least equivalent.

As used herein, the phrase "at least one of" preceding a series of items, with the term "or" to separate any of the items, modifies the list as a whole, rather than each item of the list. The phrase "at least one of" does not require selection of at least one item; rather, the phrase allows a meaning that includes at least one of any one of the items, and/or at least one of any combination of the items, and/or at least one of each of the items. By way of example, the phrase "at least one of A, B, or C" may refer to: only A, only B, or only C; or any combination of A, B, and C.

A phrase such as an "aspect" does not imply that such aspect is essential to the subject technology or that such aspect applies to all configurations of the subject technology. A disclosure relating to an aspect may apply to all configurations, or one or more configurations. An aspect may provide one or more examples. A phrase such as an aspect may refer to one or more aspects and vice versa. A phrase such as an "embodiment" does not imply that such embodiment is essential to the subject technology or that such embodiment applies to all configurations of the subject technology. A disclosure relating to an embodiment may apply to all embodiments, or one or more embodiments. An embodiment may provide one or more examples. A phrase such an embodiment may refer to one or more embodiments and vice versa. A phrase such as a "configuration" does not imply that such configuration is essential to the subject technology or that such configuration applies to all configurations of the subject technology. A disclosure relating to a configuration may apply to all configurations, or one or more configurations. A configuration may provide one or more examples. A phrase such a configuration may refer to one or more configurations and vice versa.

In one aspect, unless otherwise stated, all measurements, values, ratings, positions, magnitudes, sizes, and other specifications that are set forth in this specification, including in the claims that follow, are approximate, not exact. In one aspect, they are intended to have a reasonable range that is consistent with the functions to which they relate and with what is customary in the art to which they pertain.

To the extent that the term "include," "have," or the like is used, such term is intended to be inclusive in a manner similar to the term "comprise" as "comprise" is interpreted when employed as a transitional word in a claim.

In the Detailed Description, it can be seen that the description provides illustrative examples and the various features are grouped together in various embodiments for the purpose of streamlining the disclosure. This method of disclosure is not to be interpreted as reflecting an intention that the claimed subject matter requires more features than are expressly recited in each claim. Rather, as the following claims reflect, inventive subject matter lies in less than all features of a single disclosed configuration or .

The claims are not intended to be limited to the aspects described herein, but are to be accorded the full scope consistent with the language claims and to encompass all legal equivalents.

## Claims

1. An infusion connection line (100, 200, 300, 400, 500), comprising:
a pump infusion tube (110);
a first connector (120) coupled to a first end (112) of the pump infusion tube (110);
a second connector (130) coupled to a second end (114) of the pump infusion tube (110);
and
a directional indicator element (140) disposed between the first and second connectors (120, 130), the directional indicator element (140) disposed on a first portion of the pump infusion tube (110) and configured to indicate a correct installation direction of the pump infusion tube (110) in an infusion pump (90, 190),
wherein a second portion of the pump infusion tube (110) is configured to be disposed within the infusion pump (90, 190) and the first portion of the pump infusion tube (110) is configured to be disposed external to the infusion pump (90, 190) when the second portion is disposed within the infusion pump (90, 190), the infusion pump (90, 190) being any of a plurality of types of infusion pumps used in the health care industry, and
wherein the infusion connection line (100, 200, 300, 400, 500) is configured to couple a gravity intravenous infusion set (20) to the infusion pump (90, 190),
and wherein the directional indicator element (140) is one of formed within the material of the pump infusion tube (110) and embedded within the material of the pump infusion tube (110).

2. The infusion connection line (100, 200, 300, 400, 500) of claim 1, wherein the first connector (120) is a female luer connector.

3. The infusion connection line (100, 200, 300, 400, 500) of claim 2, wherein the first connector (120) is coupled to a first connector cap (125).

4. The infusion connection line (100, 200, 300, 400, 500) of claim 2, wherein the first connector (120) is coupled to a female stop cock assembly (150).

5. The infusion connection line (100, 200, 300, 400, 500) of claim 2, wherein the first connector (120) is coupled to a male stop cock assembly (160).

6. The infusion connection line (100, 200, 300, 400, 500) of claim 2, wherein the first connector (120) is coupled to a needle free connector (170).

7. The infusion connection line (100, 200, 300, 400, 500) of claim 2, wherein the first connector (120) is coupled to the gravity IV infusion set (20).

8. The infusion connection line (100, 200, 300, 400, 500) of claim 1, wherein the second connector (130) is a male luer connector.

9. The infusion connection line (100, 200, 300, 400, 500) of claim 8, wherein the second connector (130) is coupled to a second connector cap (135).

10. The infusion connection line (100, 200, 300, 400, 500) of claim 8, wherein the second connector (130) is coupled to an extension infusion line (180), the extension infusion line (180) configured to deliver a fluid from the infusion pump (90, 190) to a receiving entity.

11. The infusion connection line (100, 200, 300, 400, 500) of claim 1, wherein the pump infusion tube (110) has a wall thickness of 0.45 mm to 0.65 mm.

12. The infusion connection line (100, 200, 300, 400, 500) of claim 1, wherein the pump infusion tube (110) has a length of 300 mm to 400 mm.

13. The infusion connection line (100, 200, 300, 400, 500) of claim 1, wherein the pump infusion tube (110) is formed of a high elasticity material comprising one of silicone and thermoplastic polyurethane.

14. An infusion set, comprising:
the infusion connection line (100, 200, 300, 400, 500) of any of claims 1 to 13;
and
a gravity intravenous set (20) coupled to the pump infusion tube (110).

## Patentansprüche

1. Infusionsverbindungsleitung (100, 200, 300, 400, 500), aufweisend:
einem Pumpeninfusionsschlauch (110);
einem ersten Anschlussstück (120), das mit einem ersten Ende (112) des Pumpeninfusionsschlauchs (110) verbunden ist;
einem zweiten Verbinder (130), der mit einem zweiten Ende (114) des Pumpeninfusionsschlauchs (110) verbunden ist; und
ein Richtungsanzeigeelement (140), das zwischen dem ersten und dem zweiten Verbinder (120, 130) angeordnet ist, wobei das Richtungsanzeigeelement (140) an einem ersten Abschnitt des Pumpeninfusionsschlauchs (110) angeordnet und so konfiguriert ist, dass es eine korrekte Einbaurichtung des Pumpeninfusionsschlauchs (110) in einer Infusionspumpe (90, 190) anzeigt,
wobei ein zweiter Abschnitt des Pumpeninfusionsschlauchs (110) so konfiguriert ist, dass er innerhalb der Infusionspumpe (90, 190) angeordnet ist, und der erste Abschnitt des Pumpeninfusionsschlauchs (110) so konfiguriert ist, dass er außerhalb der Infusionspumpe (90, 190) angeordnet ist, wenn der zweite Abschnitt innerhalb der Infusionspumpe (90, 190) angeordnet ist, wobei die Infusionspumpe (90, 190) eine von mehreren Arten von Infusionspumpen ist, die in der Gesundheitsbranche verwendet werden, und
wobei die Infusionsverbindungsleitung (100, 200, 300, 400, 500) so konfiguriert ist, dass sie ein intravenöses Schwerkraftinfusionsset (20) mit der Infusionspumpe (90, 190) verbindet,
und wobei das Richtungsanzeigeelement (140) entweder in das Material des Pumpeninfusionsschlauchs (110) eingeformt oder in das Material des Pumpeninfusionsschlauchs (110) eingebettet ist.

2. Infusionsverbindungsleitung (100, 200, 300, 400, 500) nach Anspruch 1, wobei der erste Verbinder (120) ein weiblicher Luer-Verbinder ist.

3. Infusionsverbindungsleitung (100, 200, 300, 400, 500) nach Anspruch 2, wobei der erste Verbinder (120) mit einer ersten Verbinderkappe (125) verbunden ist.

4. Infusionsverbindungsleitung (100, 200, 300, 400, 500) nach Anspruch 2, wobei der erste Verbinder (120) mit einer weiblichen Absperrhahnbaugruppe (150) verbunden ist.

5. Infusionsverbindungsleitung (100, 200, 300, 400, 500) nach Anspruch 2, wobei der erste Verbinder (120) mit einer männlichen Absperrhahnbaugruppe (160) verbunden ist.

6. Infusionsverbindungsleitung (100, 200, 300, 400, 500) nach Anspruch 2, wobei der erste Verbinder (120) mit einem nadelfreien Verbinder (170) verbunden ist.

7. Infusionsverbindungsleitung (100, 200, 300, 400, 500) nach Anspruch 2, wobei der erste Verbinder (120) mit dem Schwerkraft-IV-Infusionsset (20) verbunden ist.

8. Infusionsverbindungsleitung (100, 200, 300, 400, 500) nach Anspruch 1, wobei der zweite Verbinder (130) ein männlicher Luer-Verbinder ist.

9. Infusionsverbindungsleitung (100, 200, 300, 400, 500) nach Anspruch 8, wobei der zweite Verbinder (130) mit einer zweiten Verbinderkappe (135) verbunden ist.

10. Infusionsverbindungsleitung (100, 200, 300, 400, 500) nach Anspruch 8, wobei der zweite Verbinder (130) mit einer Verlängerungsinfusionsleitung (180) verbunden ist, wobei die Verlängerungsinfusionsleitung (180) so konfiguriert ist, dass sie eine Flüssigkeit von der Infusionspumpe (90, 190) zu einer Empfangseinheit befördert.

11. Infusionsverbindungsleitung (100, 200, 300, 400, 500) nach Anspruch 1, wobei der Pumpeninfusionsschlauch (110) eine Wandstärke von 0,45 mm bis 0,65 mm aufweist.

12. Infusionsverbindungsleitung (100, 200, 300, 400, 500) nach Anspruch 1, wobei der Pumpeninfusionsschlauch (110) eine Länge von 300 mm bis 400 mm aufweist.

13. Infusionsverbindungsleitung (100, 200, 300, 400, 500) nach Anspruch 1, wobei der Pumpeninfusionsschlauch (110) ein hochelastisches Material aufweist, das entweder Silikon oder thermoplastisches Polyurethan umfasst.

14. Infusionsset, umfassend:
die Infusionsverbindungsleitung (100, 200, 300, 400, 500) nach einem der Ansprüche 1 bis 13; und
ein Schwerkraft-Infusionsset (20), das mit dem Pumpeninfusionsschlauch (110) verbunden ist.

## Revendications

1. Ligne de raccordement de perfusion (100, 200, 300, 400, 500), comprenant :
un tuyau de perfusion de pompe (110) ;
un premier connecteur (120) raccordé à une première extrémité (112) du tuyau de perfusion de pompe (110) ;
un deuxième connecteur (130) raccordé à une deuxième extrémité (114) du tuyau de perfusion de pompe (110) ; et
un élément indicateur de direction (140) disposé entre le premier et le deuxième connecteur (120, 130), l'élément indicateur de direction (140) étant disposé sur une première section du tuyau de perfusion de pompe (110) et conçu pour indiquer la direction de montage correcte du tuyau de perfusion de pompe (110) dans une pompe de perfusion (90, 190),
une deuxième section du tuyau de perfusion de pompe (110) étant conçue pour être disposée à l'intérieur de la pompe de perfusion (90, 190), et la première section du tuyau de perfusion de pompe (110) étant conçue pour être disposée à l'extérieur de la pompe de perfusion (90, 190) lorsque la deuxième section est disposée à l'intérieur de la pompe de perfusion (90, 190), la pompe de perfusion (90, 190) étant l'une quelconque d'une pluralité de types de pompes de perfusion utilisées dans le secteur de la santé, et
la ligne de raccordement de perfusion (100, 200, 300, 400, 500) étant conçue pour connecter un kit de perfusion intraveineuse (20) fonctionnant par gravité à la pompe de perfusion (90, 190),
et sachant que l'élément indicateur de direction (140) est formé dans le matériau du tuyau de perfusion de pompe (110) ou est intégré dans le matériau du tuyau de perfusion de pompe (110) .

2. La ligne de raccordement de perfusion (100, 200, 300, 400, 500) selon la revendication 1, sachant que le premier connecteur (120) est un connecteur à douille Luer.

3. La ligne de raccordement de perfusion (100, 200, 300, 400, 500) selon la revendication 2, sachant que le premier connecteur (120) est relié à un premier capuchon de connecteur (125) .

4. La ligne de raccordement de perfusion (100, 200, 300, 400, 500) selon la revendication 2, sachant que le premier connecteur (120) est raccordé à un ensemble de douille à robinet d'arrêt (150).

5. La ligne de raccordement de perfusion (100, 200, 300, 400, 500) selon la revendication 2, sachant que le premier connecteur (120) est raccordé à un ensemble enfichable à robinet d'arrêt (160).

6. La ligne de raccordement de perfusion (100, 200, 300, 400, 500) selon la revendication 2, sachant que le premier connecteur (120) est raccordé à un connecteur sans aiguille (170) .

7. La ligne de raccordement de perfusion (100, 200, 300, 400, 500) selon la revendication 2, sachant que le premier connecteur (120) est raccordé au kit de perfusion IV (20) fonctionnant par gravité.

8. La ligne de raccordement de perfusion (100, 200, 300, 400, 500) selon la revendication 1, sachant que le deuxième connecteur (130) est un connecteur enfichable de type Luer.

9. La ligne de raccordement de perfusion (100, 200, 300, 400, 500) selon la revendication 8, sachant que le deuxième connecteur (130) est raccordé à un deuxième capuchon de connecteur (135).

10. La ligne de raccordement de perfusion (100, 200, 300, 400, 500) selon la revendication 8, sachant que le deuxième connecteur (130) est raccordé à une ligne de perfusion d'extension (180), ladite ligne de perfusion d'extension (180) étant conçue pour transporter un fluide de la pompe de perfusion (90, 190) vers une entité réceptrice.

11. La ligne de raccordement de perfusion (100, 200, 300, 400, 500) selon la revendication 1, sachant que le tuyau de perfusion de pompe (110) a une épaisseur de paroi comprise entre 0,45 mm et 0,65 mm.

12. La ligne de raccordement de perfusion (100, 200, 300, 400, 500) selon la revendication 1, sachant que le tuyau de perfusion de pompe (110) a une longueur comprise entre 300 mm et 400 mm.

13. La ligne de raccordement de perfusion (100, 200, 300, 400, 500) selon la revendication 1, sachant que le tuyau de perfusion de pompe (110) est constitué d'un matériau hautement élastique comprenant du silicone ou un polyuréthane thermoplastique.

14. Kit de perfusion, comprenant :
la ligne de raccordement de perfusion (100, 200, 300, 400, 500) selon l'une des revendications 1 à 13 ; et
un kit intraveineux (20) fonctionnant par gravité et raccordé au tuyau de perfusion de pompe (110).
